# EUROPEAN PATENT APPLICATION

(11) **EP 1 441 301 A2**
(43) Date of publication of application: **28.07.2004**
(21) Application number: 04250295.5
(22) Date of filing: 21.01.2004
(51) Int. Cl.: G06F 19/00

(54) **Method for identifying and communicating with potential clinical trial participants**

(30) Priority: 21.01.2003 US 441493 P; 19.11.2003 US 716919
(71) Applicant: SIEMENS CORPORATE RESEARCH, INC., Princeton, New Jersey 08540 (US)
(72) Inventor: Bruschi, Paul, Princeton, NJ 08550 (US); Masticola, Stephen P., Kingstone, NJ 08528 (US); Sherman, William, Belle Meade, NJ 08502 (US)
(74) Representative: O'Connell, David Christopher

(57) **Abstract**

The present invention is a method and system for selecting clinical trial candidates. Medical records are obtained from a source of patient clinical data, and are searched by a clinical trial candidate identification service for suitability for a particular clinical trial. The privacy of patients whose medical records are searched is preserved by replacing the identity of the patient with a secure patient code before the records are released to the clinical trial candidate identification service.

## Description

### Cross Reference to Related Applications

This application claims the benefit of U.S. Provisional Application Serial No. 60/441,493, filed on January 21, 2003, which is incorporated by reference herein in its entirety.

### Field of the Invention

The present invention relates generally to the field of medical information processing systems, and more particularly, to a method for identifying and communicating with potential clinical trial participants while maintaining the privacy of those potential participants.

### Background of the Invention

Selection of candidates for clinical trials is an expensive process. It is estimated that it costs drug companies several thousand dollars for each participant selected. Furthermore, sometimes even after being selected, candidates must be dropped from a trial because of inaccurate or incorrect information. That may delay the clinical trial, causing an even greater expense.

The currently used process of finding patients for clinical trials includes gathering clinical data about prospective candidates, performing data mining to find candidates who meet the selection criteria for the trial, and recruiting the clinical trial participants from among the candidates. In the present state of the art, those steps are usually performed in an ad-hoc manner, without any central data repository and without a systematic process. One reason for that state of affairs is the concern for patients' privacy.

For example, the gathering of clinical data about prospective candidates is generally done on a small, localized scale because of concerns about patients' privacy. Sponsors of a clinical trial may advertise for candidates locally to doctors within a specific medical center or group. Doctors outside that group do not learn of the trial, even though they may have qualifying patients.

Clinical data often exists on a larger scale in computerized formats. For example, health care insurers or payment brokers maintain large databases containing exactly the data needed to search for clinical trial candidates. Patients, health care providers and insurance companies, however, are reluctant to release that data because of concerns about the privacy of the patients.

Large-scale centralized databases of patient clinical data are viewed as a weak point for patient privacy. Anyone with access to a database of clinical records according to current technology can read sensitive information about any patient in the database. Because of those privacy concerns, opportunities to integrate automated data sources, such as clinical information provided to healthcare payers for billing purposes, are not exploitable.

The acceptance of industry-standard data formats, such as the Health Level 7 (HL7) protocol for clinical data and formats for clinical trials data proposed by the Clinical Data Interchange Standards Consortium (CDISC), has made health care data more suitable for automated searching to find clinical trial candidates. That advantage, however, is lost in the current state of the art because of the privacy issues raised by such large health care databases.

There is therefore presently a need to provide a method and system for utilizing available health care information to identify clinical trial candidates. The technique should preferably maximize the protection of the patients' privacy. To the inventors' knowledge, there is currently no such technique available.

### Summary of the Invention

The present invention addresses the needs described above by providing a method for identifying clinical trial candidates. In one embodiment of the invention, which is typically performed by a data exchange service, the method includes the steps of receiving from a patient clinical data source, patient data including identities of patients, replacing the identities of the patients in the patient data with secure patient codes, forwarding the patient data with secure patient codes to a clinical trial candidate identification service, receiving from the clinical trial candidate identification service a clinical trial candidate proposal including a secure patient code corresponding to a proposed clinical trial candidate, determining an identity of the proposed clinical trial candidate from the secure patient code, and forwarding the identity of the proposed clinical trial candidate to a candidate contact.

The candidate contact may be a health care provider of the candidate, or may be the proposed clinical trial candidate himself. The step of replacing the identities of the patients in the patient data with secure patient codes may include encrypting the identities to create secure patient codes, and the step of determining an identity of the proposed clinical trial candidate comprises decrypting the secure patient code corresponding to the proposed clinical trial candidate. Alternatively that step may include replacing the identities with unique codes and maintaining a table correlating the identities with the unique codes, in which case the step of determining an identity of the proposed clinical trial candidate includes looking up in the table an identity of a patient corresponding to the secure patient code.

The method may further include the step of extracting patient medical information from the patient data received from a patient clinical data source, and may include the step of reformatting the patient data received from a patient clinical data source.

The clinical data source may be a database containing transactions between health care providers and payers, or may be a hospital network.

The method may also include the steps of receiving from the candidate contact a status of the clinical trial candidate proposal, and forwarding the status to the clinical trial candidate identification service. In that case, the status may include the identity of the proposed candidate, and the method may further comprise the step of replacing the identity of the proposed candidate with a secure patient code before forwarding the status to the clinical trial candidate identification service.

The method may also include the steps of receiving from the clinical trial candidate identification service, descriptive information about a clinical trial of the clinical trial candidate proposal, and forwarding the information to the candidate contact.

In another embodiment of the invention, typically performed by a clinical trial candidate identification service, a method is provided for identifying clinical trial candidates. The method includes the step of receiving at least one clinical data record, where each record includes clinical data and a secure patient code uniquely identifying the record without revealing an identity of a corresponding patient. A candidate selection criterion for a clinical trial is received, and the clinical data records are searched for a matching clinical data record based on the candidate selection criterion. If a matching clinical data record is found, then a contact request is forwarded including at least a secure patient code from the matching clinical data record.

In that method, the contact request may be forwarded to a trusted entity having an identity of a patient corresponding to the forwarded secure patient code. The secure patient code may be an encrypted identity of a patient, or may be a unique code corresponding to an entry in a table accessible to a trusted entity.

The method may further include the step of receiving descriptive information about the clinical trial, in which case the contact request includes the descriptive information. The clinical data records may be received from a data exchange service.

The secure patient code corresponding to a matching clinical data record may be forwarded to the data exchange service. The clinical data records may be received from an entity controlling a database containing transactions between health care providers and payers. The secure patient code corresponding to a matching clinical data record may be forwarded to an entity controlling a database containing transactions between health care providers and payers

The method may also include the steps of maintaining records of matching clinical data records, and forwarding a contact request including a secure patient code only if that code has not already been forwarded.

In yet another embodiment of the invention, a method is provided for selecting clinical trial candidates. That method includes the step of periodically receiving clinical data records, each of the records including clinical data and a secure patient code uniquely identifying the record without revealing an identity of a corresponding patient. The method also includes the step of periodically searching the data records to identify records of clinical trial candidates.

A system for identifying clinical trial candidates is provided in another embodiment of the invention. That system comprises a data exchange service for receiving patient records from a patient clinical data source and replacing identities of patients in each patient record with a secure patient code, and a clinical trial candidate identification service for receiving the patient records with secure patient codes from the data exchange service, and identifying a patient record as a clinical trial candidate.

The clinical trial candidate identification service of that system may forward a secure patient code of the identified patient record to the data exchange service, in which case the data exchange service determines the identity of the patient for contacting the patient. The data exchange service may receive a status of the clinical trial candidate proposal, replace an identity of the proposed candidate with a secure patient code; and forward the status to the clinical trial candidate identification service.

The clinical trial candidate identification service of the system may further be for receiving candidate selection criteria for a clinical trial, in which case the clinical trial candidate identification service identifies a patient record based on the candidate selection criteria.

The data exchange service of the system may replace the identities of the patients with secure patient codes that are encryptions of the identities of the patients. The data exchange service may alternatively replace the identities of the patients with secure patient codes that are unique codes, in which case the data exchange service further comprises a lookup table correlating each unique code with a patient code.

The data exchange service of the system may further be for extracting patient medical information from the patient data received from a patient clinical data source, or for reformatting the patient data received from a patient clinical data source. The clinical data source may be a database containing transactions between health care providers and payers, or may be a hospital network.

### Brief Description of the Drawings

FIG. 1 is a schematic diagram showing the functional elements of a system according to one embodiment of the invention.

FIG. 2 is a schematic diagram showing a data exchange service according to one embodiment of the invention.

FIG. 3 is a schematic diagram showing a clinical trial candidate identification service according to one embodiment of the invention.

FIG. 4 is a time line showing a sequence of events according to a method of one embodiment of the invention.

FIG. 5 is a block diagram showing a method in accordance with one embodiment of the invention.

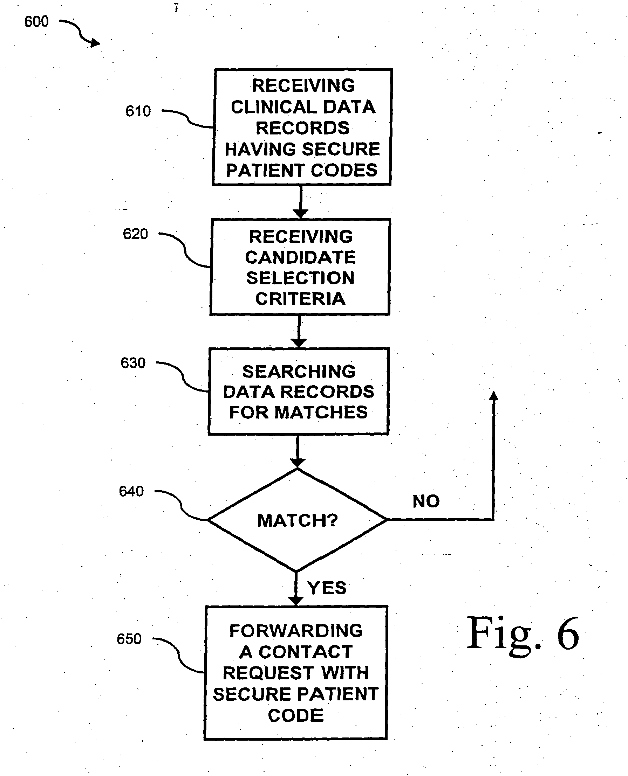

FIG. 6 is a block diagram showing a method in accordance with another embodiment of the invention.

FIG. 7 is a schematic diagram showing organizational relationships of the various components of the invention according to one embodiment.

FIG. 8 is a schematic diagram of an exemplary implementation according to one embodiment of the invention.

### Description of the Invention

The present invention integrates the business processes of separate organizations to reduce the effort necessary to find volunteers for clinical trials, while reducing the probability that confidential clinical data will be released to unauthorized persons. One aspect of the invention is that it provides a new service to communicate to potential clinical trial participants while providing improved adherence to the privacy requirements of the Health Insurance Portability and Accountability Act of 1996 (HIPAA).

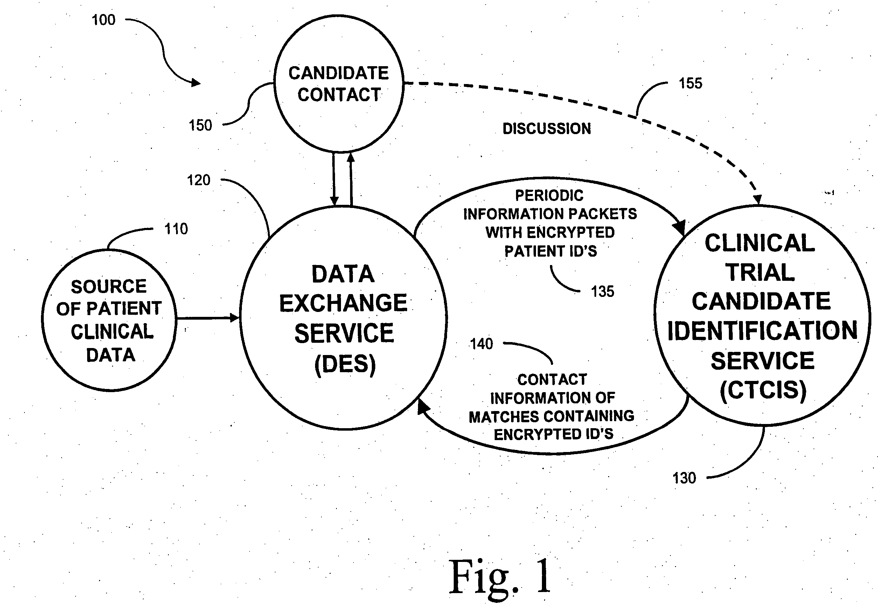

As shown in FIG. 1, three communicating parts are integrated in the system 100 of the present invention: a source of patient clinical data 110, a data exchange service (DES) 120, and a clinical trial candidate identification service (CTCIS) 130. Patient clinical history data (e.g., test results and diagnoses) is collected by the source of patient clinical data 110 and periodically forwarded in a predetermined format to the data exchange service 120, which stores it in a database. The DES 120 performs any necessary format and medical vocabulary standardization on the incoming clinical data. The data exchange service periodically extracts data from the database and constructs appropriately formatted information packets. Those information packets 135 are sent to the clinical trial candidate identification service 130 to be stored in its clinical history database. The DES replaces the patient identification and contact information with a secure identifier such as an encrypted version of that information to ensure privacy. The clinical trial candidate identification service 130 performs a periodic search or data mining operation on the clinical history database to find candidate patients for clinical trials. When a match is found, the encoded identification and contact information 140 are sent back to the data exchange service so that it can be used to contact a candidate contact 150 such as a healthcare professional, possibly the patient's physician, or the candidate himself. The DES uses the secure identifier to determine the identity of the trials candidate.

The term "match" as used herein means that candidate characteristics sought for a particular trial are best matched by the clinical data of one or more of the patients in the database. An exact match is not necessary. One technique for selecting prospective candidates is described in United States Patent Application Publication No. 2003/0130871, published on July 10, 2003, and assigned to the same assignee as the present application. The contents of that document are hereby incorporated by reference in their entirety herein.

The number of people authorized to view the unencoded patient identification is limited. Details about the search criteria used to identify the candidate patient are not included in the initial communication 140. That is to guarantee the separation of information so that it is impossible for unauthorized persons to link a specific patient with a specific medical condition. The healthcare professional or other candidate contact 150 is requested to contact the clinical trial candidate identification service 130 directly for more information about the clinical trial. A discussion 155 between the clinical trial candidate identification service 130 and the candidate contact 150 would determine if it is appropriate to contact the patient to see if he or she is willing to participate in the clinical trial.

The present invention utilizes clinical data obtained as a byproduct of other business processes for use in seeking out patients for clinical trials. To protect the privacy of patients, the invention maintains the data of the patients in such a way that the identity of the patient is only co-located with the patient's clinical data in those organizations that are authorized to know both of those pieces of information (e.g., the patient's hospital or health maintenance organization). That makes it much more difficult to deliberately or accidentally reveal sensitive patient information to unauthorized personnel.

The DES 120 must be trusted by the patient and the organizations already involved in his treatment, because it is given the patient identification and the clinical data. However, the CTGIS 130 need not be trusted since it is given encoded identification information.

The DES 120 encodes all patient identification information such as name, address, social security number, etc. (patient clinical information is kept unencrypted, so that the CTCIS 130 can perform data mining on it). The encoding may be in the form of encrypting or performing a one-way hash. The encoded patient identification information can then be stored in the CTCIS but cannot be read at the CTCIS because the keys needed to decrypt that data are kept in the DES and are secret from the CTCIS. When the CTCIS identifies a clinical trial candidate, the encoded patient identification information is then sent back to the DES. The DES decrypts the patient identification information so that the candidate contact 150, preferably either the patient or healthcare provider, can be contacted. Thus, the patient's medical history will remain anonymous until the CTCIS has identified the patient as a possible clinical trial candidate and the patient and healthcare provider have been contacted.

In an alternative embodiment, the DES 120 encodes the patient identification information by stripping it from the clinical documents, retaining that information in its own database, substituting a unique identifier, and sending the clinical documents and unique identifier to the CTCIS 130. When a candidate is found, the unique identifier and metadata on the trial are sent back to the DES. The DES looks up the patient identification information and the patient is contacted.

In another embodiment, the DES 120 is never given the identity of the patient. Instead, the health care provider supplies its own unique patient identifier to the DES, rather than patient identity and contact information. Rather than contacting the patient directly, in that embodiment the DES contacts the health care provider and sends the unique identifier, plus protocol information for the clinical trial in an agreed format. The healthcare provider then administers the trial. An advantage of that embodiment is that the DES need not be trusted with the identity of any participant. That gives the patient an additional degree of privacy.

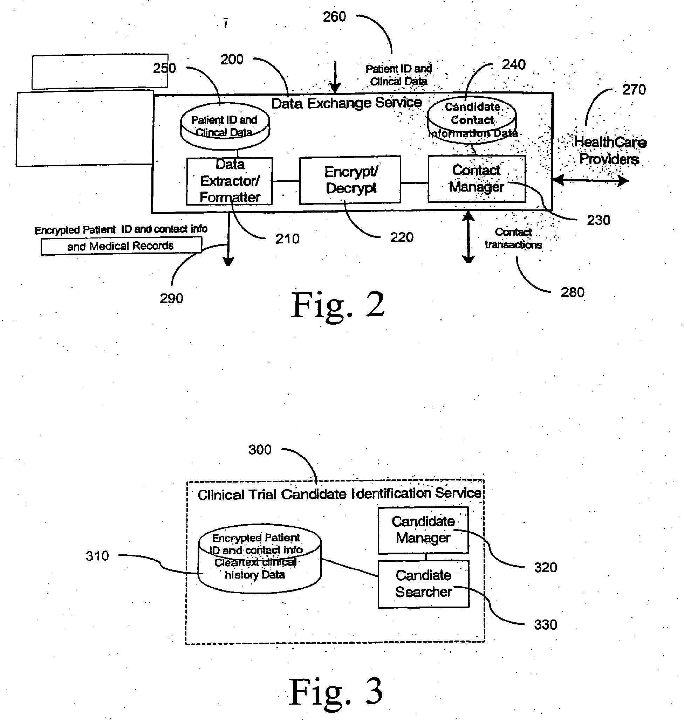

The data exchange service 200, shown in FIG. 2, may include at least five components. A patient ID and clinical data database 250 temporarily holds patient ID and contact information as well as the clinical history data. That data is received through an interface 260 with a source of patient clinical data, and is first processed by a data extractor/formatter 210.

The data extractor/formatter 210 operates on data from the source of patient clinical data 260 and extracts patient medical information (patient data and medical records) that could be of interest in searching for clinical trial candidates. That information may be appropriately formatted (e.g., Clinical Data Interchange Standards Consortium (CDISC) format) when it is transmitted to the CTCIS. The data extractor uses an encryptor/decryptor 220 to encrypt the patient ID and contact information.

The encryptor/decryptor 220 encrypts the patient ID and contact information for privacy. The medical records of the patient are not encrypted. The CTCIS is not permitted to read the patient ID or contact information. To that end, the encryptor specially tags the patient data so that the decryptor can later decrypt it. The encrypted patient contact information and the medical records are sent through the interface 290 to the CTCIS so that they can be stored at the CTCIS.

The data exchange service 200 receives contact requests 280 from the clinical trial candidate identification service to contact candidate patients. A contact manager 230 in the DES saves the patient contact information in a candidate contact information database 240 and initiates a communication 270 with a patient contact such as the patient's healthcare provider (e.g., physician, medical center). The candidate contact information database 240 maintains the list of candidate patients and the status of the inquiry. The contact manager 230 also handles status queries from the CTCIS. The responses might be, for example, No Response, Accept, or Decline.

The clinical trial candidate identification service (CTCIS) 300, shown in detail in FIG. 3, may be part of a clinical trial maintenance organization, described below. The CTCIS includes a clinical history database 310 that contains the encrypted patient ID and contact information as well as the clear (unencoded) text clinical history data.

A candidate searcher 330 applies specific search criteria periodically to the data stored in the clinical history database 310 to identify candidate patients for a specific clinical trial. When a possible clinical trial candidate is found, the encrypted candidate ID and contact information is passed from the candidate searcher 330 to the candidate manager 320.

The candidate manager 320 determines whether the proposed candidate has already been proposed for a particular clinical trial. If not, then a unique identifier is created for the candidate patient. The encrypted patient ID and contact information and the unique candidate ID are sent to the contact manager 230 in the data exchange service 200 (FIG. 2). The candidate manager 320 then periodically queries the contact manager 230 for a status of the contact.

In an alternative embodiment, the candidate manager 320 transfers only contact information related to the clinical trial. In that case, the role of the contact manager 230 within the DES 200 (FIG. 2) is limited to actually contacting the patient or healthcare provider and providing information about how to contact the administrator of the clinical trial. For example, the contact manager 230 may provide a phone number or Web site with a login and password specifically set up for the candidate to browse. The Web site preferably contains information pertaining to the particular clinical trial and the particular candidate such as why the candidate was chosen. In that way, there could be a total separation from the clinical trial information and the patient identification throughout the process of choosing and contacting the candidates.

One principle of the present invention that assures a patient's privacy is the guarantee that no unauthorized person can determine a patient's identity and medical condition at the same time. When the medical history is sent to the CTCIS, all identity and contact information is encoded. That makes it impossible for a person with access to the CTCIS to identify the patient. In order to contact the candidate, it is necessary to send the encrypted ID and contact information back to the Data Exchange Service. An observer of that transaction would not be able to identify the patient or determine why the patient is being contacted. When the patient ID information is decrypted, the identity of the patient is known, but there is no indication of why the patient has been selected as a candidate for a clinical trial. It is only when a trusted patient contact such as the patient's healthcare professional contacts the CTCIS directly that the patient contact will have any further information available about the selection of the patient as a candidate. Only at that point will information about the proposed clinical trial be available.

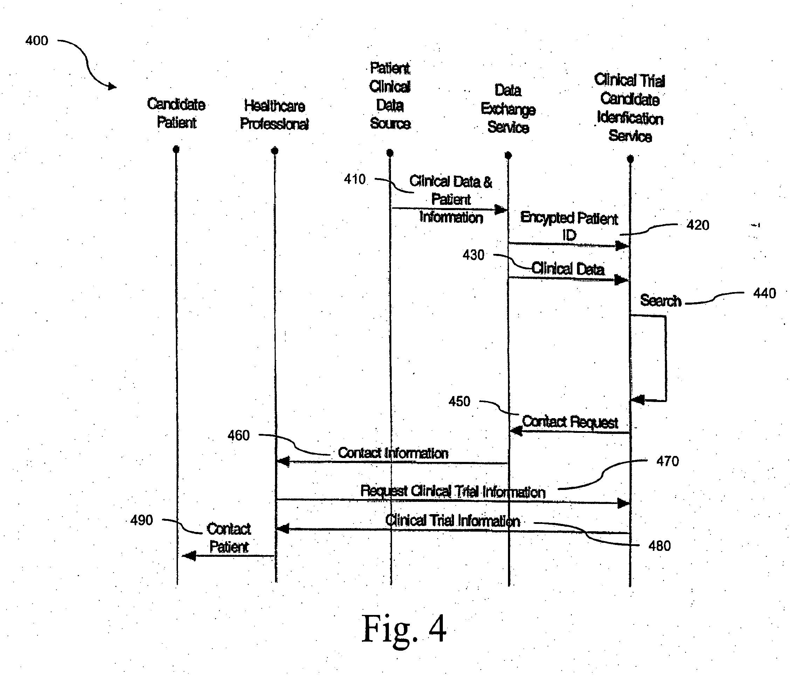

FIG. 4 shows a typical sequence 400 of data exchange among the various services and parties involved. That sequence 400 starts with the patient and medical data 410 from the patient clinical data source. Those data are transferred to the data exchange service where the patient ID data are encrypted. The encrypted patient ID data 420 and the clinical data 430 are transferred to the clinical trial candidate identification service. The clinical data are searched 440 to identify possible clinical trial candidates. When a candidate is identified, a contact request 450 is sent back to the data exchange service. That contact request provides enough information so that the patient can be identified within the data exchange service, while the CTCIS has no detailed contact information. Contact information 460 is sent on to the candidate contact (healthcare professional) informing the contact that a patient may qualify for a clinical trial and giving specific contact information at the CTCIS. Once the healthcare professional contacts the CTCIS with a request for trial information 470, further details 480 about the clinical trial can be given. Later, the patient can be contacted 490 by the healthcare professional for further consultation.

Patient privacy is maintained throughout the identification and contact phase of a clinical trial. It is also possible that the CTCIS may never know the true identity of any of the trial participants.

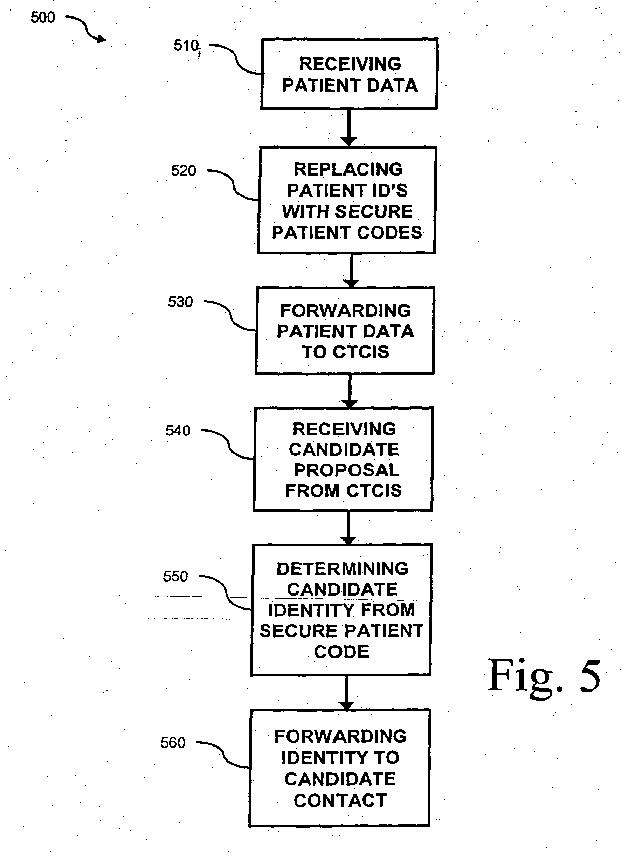

A method 500 performed by a data exchange service (DES) according to one embodiment of the invention is shown in FIG. 5. The DES initially receives patient data (step 510) from a patient clinical data source. That source may be a database containing transactions between health care providers and payers, or may be a hospital network or other healthcare provider network. One advantage of the present invention is that patient clinical data in standardized formats exists in such data sources today.

The identities of patients in the patient data are then replaced (step 520) by secure patient codes. The secure patient codes may be encrypted versions of the data itself, in which case the DES holds the encryption key, and only the DES is able to decrypt that information. In another embodiment, as described above, the secure patient codes may be unique codes that are stored in a look-up table in the DES. In that case as well, only the DES has the information necessary to determine an identity of a patient. In addition to replacing the patient ID, the DES may extract patient medical information from the patient data and may reformat the patient data.

The DES then forwards (step 530) the patient data with the secure patient codes to a clinical trial candidate identification service (CTCIS), as described above. After the CTCIS processes the information and identifies potential clinical trial candidates, the DES receives (step 540) from the CTCIS a candidate proposal. The proposal includes the secure patient code corresponding to the patient selected by the CTCIS as a clinical trial candidate.

Once the DES receives a candidate proposal, it determines (step 550) the identity of the clinical trial candidate from the secure patient code. That determination may be made by decrypting an encrypted patient identity, or by looking up a unique identifier code in a secure lookup table within the DES.

The patient identity is then forwarded (step 560) to a candidate contact. The contact may, for example, be a health care provider, or may be the proposed clinical trial candidate himself. The DES may then receive from the contact a status of the proposal, and forward that status to the CTCIS. If the status includes the identity of the candidate, the DES may encode that identity before forwarding the status to the CTCIS.

Another method 600 according to the invention is performed by the CTCIS. In that method, clinical data records are received (step 610). Each clinical data record contains clinical data and a secure patient code that uniquely identifies the record without revealing the identity the corresponding patient, as described above.

The CTCIS also receives (step 620) a candidate selection criterion for a clinical trial. That criterion may be received, for example, from a clinical trial data manager that manages clinical trials and maintains clinical trial data.

The clinical data records are then searched (step 630), using the candidate selection criterion as a search parameter. If a match is found (step 640), then a contact request is forwarded (step 650), including at least a secure patient code from the matching data record. As described above, the identity of the candidate is determined from the secure patient code by a trusted entity outside the CTCIS. The contact request may include descriptive information about the trial that can be used by the candidate or the heath care provider in deciding whether to participate.

The organization of the communicating entities described herein may take several forms. The data exchange service, for example, may be interested in purchasing a stream of healthcare transactions from a health care insurance claims brokerage, or from other sources. The CTCIS may be funded by organizations having a vested interest in the results of the clinical trials, such as pharmaceutical companies. The CTCIS may wish to purchase packets of properly formatted patient history for inclusion their database. The CTCIS may also purchase candidate proposals from the DES.

Healthcare professionals may be compensated for dealing with candidate patients, and possibly for running clinical trials. Patients may be compensated for participating in clinical trials.

The DES and CTCIS may be deployed across or within corporate or organizational boundaries in several embodiments. In the preferred embodiments of the invention, the DES is itself a part of the organization that provides the data to the CTCIS. In those embodiments, the DES is in the same organization that already has the clinical data, so there is no problem of transmitting sensitive data to an untrusted party. The invention therefore provides the ability to form a centralized repository for selecting clinical trials patients without risking the privacy of the participants.

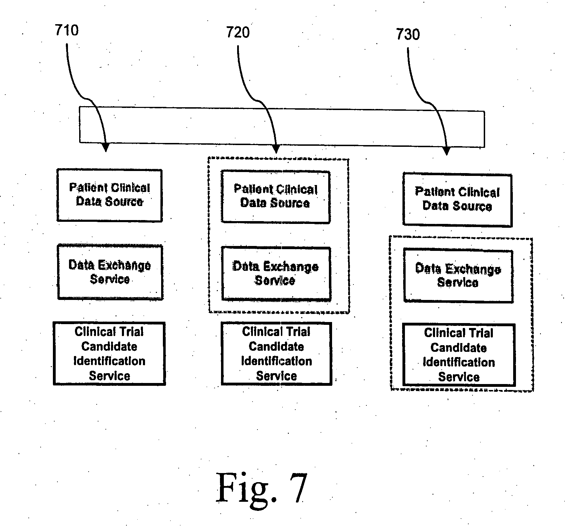

Figure 7 shows three possible organizational configurations of the present invention. Configuration 710 shows that the patient clinical data source, data exchange service, and clinical trial candidate identification service as separate organizations. Configuration 720 shows the CTCIS as a separate organization from the patient clinical data source and data exchange service, which are in a single organization. Finally configuration 730 shows the patent clinical data source as a separate organization from the CTCIS and data exchange service. The choice as to which embodiment to choose is based on the business model.

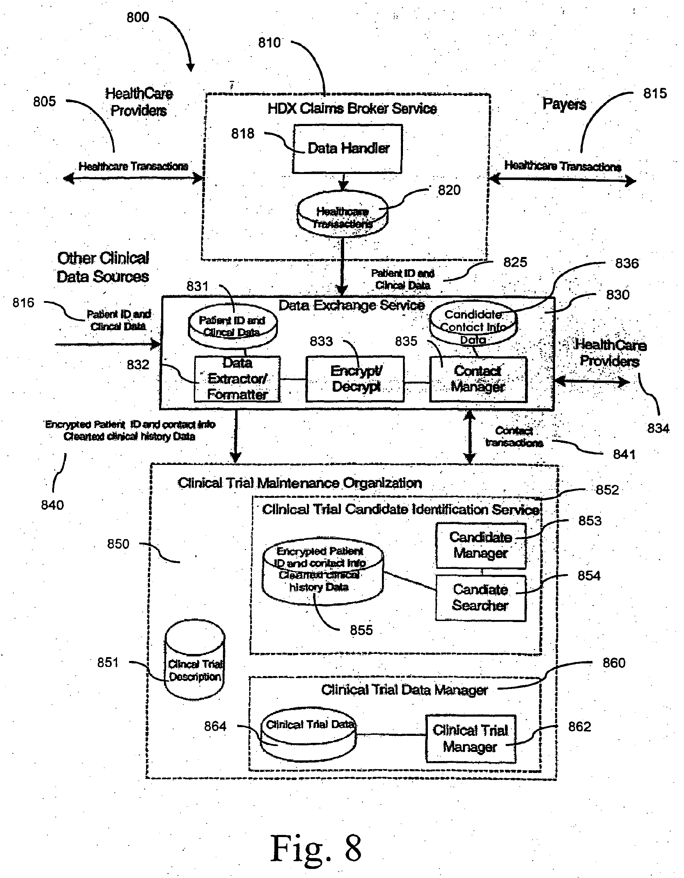

An example embodiment 800 of the present invention as it may be integrated with other systems is shown in FIG. 8. In that example, the system of the present invention is integrated with a Healthcare Data Exchange (HDX) system 810 and a Clinical Trials Maintenance Organization (CTMO) 850. Both are projects under development by Siemens Corporation.

HDX is a current project involving the communication between healthcare providers and payers. Transactions between those parties use the HL7 protocol. When completed, HDX will maintain and transfer sensitive patient information that would be highly useful for clinical trials. Despite the fact that HDX would be a useful data source, patient privacy concerns must be overcome for such a program to be workable.

The CTMO is a proposed new business organization. The concept of the overall CTMO system is to create a wide business network containing hospitals and healthcare organizations that cooperate to identify patients who are suitable for clinical trials and to administer data collection and analysis for those trials.

In the present exemplary embodiment, the HDX 810 acts as an automated source of clinical data and the CTMO 850 as its client. The HDX 810 handles healthcare transactions with health care providers 805 and with heath care payers 815, thereby having access to a large amount of heath care information. A data handler 818 maintains a database 820 of such transactions, from which is obtains patient ID and clinical data 825, which it forwards to a DES 830.

The DES 830 receives clinical data 825 from the HDX 810, as well as other clinical data from other sources 816, such as from health care providers themselves. As described above, the DES includes a data extractor/formatter 832 with a clinical data database 831, an encryptor/decryptor 833 and a contact manager with candidate contact information database 836. The DES interfaces with healthcare providers 834 or other patient contacts.

The DES sends encrypted patient ID and contact information 840, together with clear clinical history data, to the CTMO 850. The CTMO includes a CTCIS 852, together with a clinical trial data manager 860 and a clinical trial description database 851. As described above, the CTCIS 852 comprises a candidate searcher 854 with access to a clinical history database 855, and a candidate manager 853. Although the clinical trial description database is shown as part of the CTMO organization, it may be placed outside that organization. The clinical trial description database describes the clinical trial. That information is appropriately formatted (e.g., in CDISC format).

In the same CTMO 650 is the clinical trial data manager 860, including a clinical trial manager 862 and a database 864. The clinical trial data manager manages and contains the clinical trial data. That information is appropriately formatted (e.g., CDISC). Searching is therefore carried out by the same organization that manages the trials, while maintaining the anonymity of the potential participants.

Using the structure of the exemplary embodiment, existing data sources, such as a HDX between healthcare payers and providers, could provide a large amount of already available clinical history data for the CTCIS. That would be a byproduct of storing data transactions between healthcare providers and payers in the HDX. Such data is of high quality since it is very accurate and timely because providers and payers are constantly verifying that the data are correct.

The foregoing Detailed Description is to be understood as being in every respect illustrative and exemplary, but not restrictive, and the scope of the invention disclosed herein is not to be determined from the Description of the Invention, but rather from the Claims as interpreted according to the full breadth permitted by the patent laws. For example, while the method of encoding the identity of the candidate is described in terms of encrypting or substituting a unique identifier, one skilled in the art will recognize that other techniques for anonymizing the data may be used, while remaining within the scope of the invention. It is to be understood that the embodiments shown and described herein are only illustrative of the principles of the present invention and that various modifications may be implemented by those skilled in the art without departing from the scope and spirit of the invention.

## Claims

1. A method for identifying clinical trial candidates, the method comprising the steps of:
receiving from a patient clinical data source, patient data including identities of patients;
replacing the identities of the patients in the patient data with secure patient codes;
forwarding the patient data with secure patient codes to a clinical trial candidate identification service;
receiving from the clinical trial candidate identification service a clinical trial candidate proposal including a secure patient code corresponding to a proposed clinical trial candidate;
determining an identity of the proposed clinical trial candidate from the secure patient code; and
forwarding the identity of the proposed clinical trial candidate to a candidate contact.

2. The method of claim 1, wherein the candidate contact is a health care provider of the candidate.

3. The method of claim 1, wherein the candidate contact is the proposed clinical trial candidate.

4. The method of claim 1, wherein the step of replacing the identities of the patients in the patient data with secure patient codes comprises encrypting the identities to create secure patient codes, and the step of determining an identity of the proposed clinical trial candidate comprises decrypting the secure patient code corresponding to the proposed clinical trial candidate.

5. The method of claim 1, wherein the step of replacing the identities of the patients in the patient data with secure patient codes comprises replacing the identities with unique codes and maintaining a table correlating the identities with the unique codes, and the step of determining an identity of the proposed clinical trial candidate comprises looking up in the table an identity of a patient corresponding to the secure patient code.

6. The method of claim 1, further comprising the step of extracting patient medical information from the patient data received from a patient clinical data source.

7. The method of claim 1, further comprising the step of reformatting the patient data received from a patient clinical data source.

8. The method of claim 1, wherein the clinical data source is a database containing transactions between health care providers and payers.

9. The method of claim 1, wherein the clinical data source is a hospital network.

10. The method of claim 1, further comprising the steps of:
receiving from the candidate contact a status of the clinical trial candidate proposal; and
forwarding the status to the clinical trial candidate identification service.

11. The method of claim 10, wherein the status includes the identity of the proposed candidate, and the method further comprises the step of replacing the identity of the proposed candidate with a secure patient code before forwarding the status to the clinical trial candidate identification service.

12. The method of claim 1, further comprising the steps of:
receiving from the clinical trial candidate identification service, descriptive information about a clinical trial of the clinical trial candidate proposal; and
forwarding the information to the candidate contact.

13. A method for identifying clinical trial candidates, the method comprising the steps of:
receiving at least one clinical data record, each said record including clinical data and a secure patient code uniquely identifying the record without revealing an identity of a corresponding patient;
receiving a candidate selection criterion for a clinical trial;
searching the at least one clinical data record for a matching clinical data record based on the candidate selection criterion; and
if a matching clinical data record is found, then forwarding a contact request including at least a secure patient code from the matching clinical data record.

14. The method of claim 13, wherein the contact request is forwarded to a trusted entity having an identity of a patient corresponding to the forwarded secure patient code.

15. The method of claim 13, wherein the secure patient code is an encrypted identity of a patient.

16. The method of claim 13, wherein the secure patient code is a unique code corresponding to an entry in a table accessible to a trusted entity.

17. The method of claim 13, further comprising the step of receiving descriptive information about the clinical trial, and wherein the contact request includes the descriptive information.

18. The method of claim 13, wherein the at least one clinical data record is received from a data exchange service.

19. The method of claim 18, wherein the secure patient code corresponding to a matching clinical data record is forwarded to the data exchange service.

20. The method of claim 13, wherein the at least one clinical data record is received from an entity controlling a database containing transactions between health care providers and payers.

21. The method of claim 13, wherein the secure patient code corresponding to a matching clinical data record is forwarded to an entity controlling a database containing transactions between health care providers and payers.

22. The method of claim 13, further comprising the step of:
maintaining records of matching clinical data records; and
wherein the step of forwarding a contact request including a secure patient code is performed only if that code has not already been forwarded.

23. A method for selecting clinical trial candidates, the method comprising the steps of:
periodically receiving clinical data records, each said record including clinical data and a secure patient code uniquely identifying the record without revealing an identity of a corresponding patient;
periodically searching the data records to identify records of clinical trial candidates.

24. A system for identifying clinical trial candidates, comprising:
a data exchange service for receiving patient records from a patient clinical data source and replacing identities of patients in each patient record with a secure patient code;
a clinical trial candidate identification service for receiving the patient records with secure patient codes from the data exchange service, and identifying a patient record as a clinical trial candidate.

25. The system of claim 24, wherein the clinical trial candidate identification service forwards a secure patient code of the identified patient record to the data exchange service, and the data exchange service determines the identity of the patient for contacting the patient.

26. The system of claim 24, wherein the data exchange service receives a status of the clinical trial candidate proposal, replaces an identity of the proposed candidate with a secure patient code; and forwards the status to the clinical trial candidate identification service.

27. The system of claim 24, wherein the clinical trial candidate identification service is further for receiving candidate selection criteria for a clinical trial, and the clinical trial candidate identification service identifies a patient record based on the candidate selection criteria.

28. The system of claim 24, wherein the data exchange service replaces the identities of the patients with secure patient codes that are encryptions of the identities of the patients.

29. The system of claim 24, wherein the data exchange service replaces the identities of the patients with secure patient codes that are unique codes, and the data exchange service further comprises a lookup table correlating each unique code with a patient code.

30. The system of claim 24, wherein the data exchange service is further for extracting patient medical information from the patient data received from a patient clinical data source.

31. The system of claim 24, wherein the data exchange service is further for reformatting the patient data received from a patient clinical data source.

32. The system of claim 24, wherein the clinical data source is a database containing transactions between health care providers and payers.

33. The system of claim 24, wherein the clinical data source is a hospital network.
